# FASCICULE DE BREVET EUROPEEN

(11) **EP 2 694 026 B1**
(45) Date de publication et mention de la délivrance du brevet: **07.02.2018**
(21) Numéro de dépôt: 12717414.2
(22) Date de dépôt: 29.03.2012
(51) Int. Cl.: A61K 8/37, A61Q 15/00

(54) **PROCEDE COSMETIQUE DE TRAITEMENT DES ODEURS CORPORELLES HUMAINES UTILISANT UN COMPOSE 4-(3-ETHOXY-4-HYDROXYPHENYL)ALKYLCETONE OU 2-ETHOXY 4-HYDROXYALKYL PHENOL**
KOSMETISCHES VERFAHREN ZUR BEHANDLUNG VON KÖRPERGERÜCHEN UNTER VERWENDUNG EINES 4- (3-ETHOXY-4-HYDROXYPHENYL)ALKYLKETON- ODER 2-ETHOXY-4-HYDROXYALKYLPHENOLVERBINDUNG
COSMETIC METHOD FOR TREATING HUMAN BODY ODORS USING A 4-(3-ETHOXY-4-HYDROXYPHENYL)ALKYLKETONE OR 2-ETHOXY 4-HYDROXYALKYLPHENOL COMPOUND

(30) Priorité: 01.04.2011 FR 1152795; 04.04.2011 US 201161471441 P
(43) Date de publication de la demande: 12.02.2014
(73) Titulaire: L'Oréal, 75008 Paris (FR)
(72) Inventeur: DALKO, Maria, F-78000 Versailles (FR)
(74) Mandataire: Miszputen, Laurent
(86) Numéro de dépôt international: PCT/FR2012/050669
(87) Numéro de publication internationale: WO 2012/131266

(56) Documents cités:
- WO-A1-98/30201
- WO-A1-2011/039445
- JP-A- 2002 255 774
- US-B1- 6 488 919

## Description

La présente invention a donc pour objet un procédé cosmétique pour traiter les odeurs corporelles humaines en particulier les odeurs axillaires consistant à appliquer sur les matières kératiniques humaines une composition contenant dans un milieu cosmétiquement acceptable au moins un composé 4-(3-éthoxy-4-hydroxyphényl)alkylcétone ou 2-éthoxy 4-hydroxyalkylphénol de formule (I).

L'invention concerne également l'utilisation cosmétique d'au moins un composé 4-(3-éthoxy-4-hydroxyphényl)alkylcétone ou 2-éthoxy 4-hydroxyalkylphénol de formule (I) comme actif déodorant.

Dans le domaine cosmétique, il est bien connu d'utiliser en application topique, des produits déodorants contenant des substances actives de type bactéricide pour diminuer, voire supprimer, les odeurs axillaires généralement désagréables.

La sueur eccrine ou apocrine est peu odorante lorsqu'elle est sécrétée. C'est sa dégradation par les bactéries via des réactions enzymatiques qui produit des composés malodorants. Les actifs déodorants ont pour fonction de diminuer ou d'empêcher la formation des mauvaises odeurs. Les différents systèmes proposés jusqu'à présent peuvent être regroupés en grandes familles. Parmi celles-ci, il y a les substances bactéricides qui détruisent la flore bactérienne résidente. La plus employée est le Triclosan. Il y a aussi les substances qui diminuent la croissance des bactéries. Parmi ces substances, on peut citer les chélatants de métaux de transition comme l'acide éthylène diamine tetra acétique (EDTA) ou le l'acide Diéthylènetriamine pentaacétique (DPTA).

Cependant ces différents traitements appliqués sur la peau des aisselles ont tendance à provoquer des altérations de la peau se traduisant par des irrégularités, des inhomogénéités du type tache pigmentaire en particulier sur les peaux asiatiques, dyschromie ou bien encore points noirs souvent dus à la repousse du poil.

Il subsiste donc le besoin de trouver de nouveaux actifs déodorants qui soient efficaces sans les inconvénients évoqués auparavant.

La Demanderesse a découvert d'une manière surprenante et inattendue qu'en utilisant dans une composition au moins un composé 4-(3-éthoxy-4-hydroxyphényl)alkylcétone ou 2-éthoxy 4-hydroxyalkylphénol de formule (I) dont on donnera la définition plus loin, on pouvait obtenir une activité déodorante efficace sans les inconvénients énoncés précédemment.

Cette découverte constitue la base de l'invention.

La présente invention a donc pour objet un procédé cosmétique pour traiter les odeurs corporelles humaines en particulier les odeurs axillaires consistant à appliquer sur les matières kératiniques humaines une composition contenant dans un milieu cosmétiquement acceptable au moins un composé 4-(3-éthoxy-4-hydroxyphényl)alkylcétone ou 2-éthoxy 4-hydroxyalkylphénol de formule (I) que l'on définira plus loin en détail.

L'invention concerne également l'utilisation cosmétique d'au moins un composé 4-(3-éthoxy-4-hydroxyphényl)alkylcétone ou 2-éthoxy 4-hydroxyalkylphénol de formule (I) que l'on définira plus loin en détail comme actif déodorant.

L'invention concerne également une composition conditionnée
(i) sous forme pressurisée dans un dispositif aérosol ou dans un flacon pompe ;
(ii) dans un dispositif muni d'une paroi ajourée notamment une grille ;
(iii) dans un dispositif muni d'un applicateur à billes ("roll-on) ;
(iv) sous forme de bâtonnet (sticks),
(v) sous forme de poudre libre ou compactée, caractérisée par le fait qu'elle contient dans un milieu cosmétiquement acceptable au moins un composé de formule (I). Ladite composition peut comprendre en plus au moins un actif anti-transpirant et/ou au moins un actif déodorant additionnel.

L'invention concerne également une composition comprenant en plus au moins un actif anti-transpirant et/ou au moins un actif déodorant additionnel.

Selon une forme particulière, les compositions de l'invention ne comprendront pas d'huile essentielle.

Selon une autre forme particulière, les compositions de l'invention seront différentes d'une composition comprenant au moins une huile essentielle et de 0,5 à 5% d'au moins un composé 4-(3-éthoxy-4-hydroxyphényl)alkylcétone de formule (I).

Par « composition ne comprenant pas d'huile essentielle », on entend par comprenant moins de 0,01% en poids d'huile essentielle par rapport au poids total de la composition voire exempte d'huile essentielle.

D'autres objets de l'invention apparaîtront dans la suite de la description.

Par " milieu cosmétiquement acceptable", on entend milieu compatible avec la peau et/ou ses phanères ou muqueuses qui présente une couleur, une odeur et un toucher agréables et qui ne génère pas d'inconforts inacceptables (picotements, tiraillements, rougeurs), susceptibles de détourner la consommatrice d'utiliser cette composition.

Par « matières kératiniques humaines », on entend la peau (corps, visage, contour des yeux), cheveux, cils, sourcils, poils, ongles, lèvres, muqueuses.

Selon la définition donnée dans la norme internationale ISO 9235 et adoptée par la Commission de la Pharmacopée Européenne, une huile essentielle est un produit odorant généralement de composition complexe, obtenu à partir d'une matière première végétale botaniquement définie, soit par entraînement à la vapeur d'eau, soit par distillation sèche, soit par un procédé mécanique approprié sans chauffage (Expression à froid). L'huile essentielle est le plus souvent séparée de la phase aqueuse par un procédé physique n'entraînant pas de changement significatif de la composition.

Les composés 4-(3-éthoxy-4-hydroxyphényl)alkylcétones ou 2-éthoxy 4-hydroxyalkylphénols de formule (I) conformes à l'invention répondent à la formule générale (I) suivante : 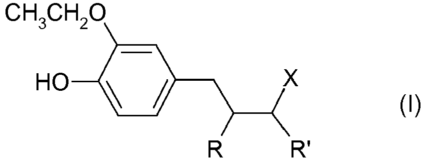 dans laquelle :
- R représente un atome d'hydrogène, ou un radical hydrocarboné, saturé ou insaturé (alkyle ou alcényle), linéaire ou ramifié, en C₁-C₆ ;
- R' représente un radical hydrocarboné, saturé ou insaturé (alkyle ou alcényle), linéaire ou ramifié, en C₁-C₁₈, éventuellement substitué par un groupe hydroxyle;
- C-X représente C=O ou CH-OH.

De préférence, R représente H, méthyle ou éthyle.

De préférence, R' représente un radical hydrocarboné linéaire, saturé en C₁-C₆ ou insaturé en C₂-C₆, éventuellement substitué par un groupe hydroxyle.

De préférence, les composés répondent à la formule (I), dans laquelle :
- lorsque - C-X représente C=O, R désigne hydrogène et R' représente un radical alkyle linéaire en C₁-C₆, éventuellement substitué par un OH et plus préférentiellement R' désigne méthyle ou éthyle; ou bien
- lorsque - C-X représente CH-OH, R désigne hydrogène et R' représente un radical alkyle linéaire en C₁-C₆, éventuellement substitué par un OH et plus préférentiellement R' désigne méthyle ou éthyle.

On peut bien évidemment utiliser un mélange de composés de formule (I).

On peut citer de préférence les composés (1), (2) et (3) suivants : 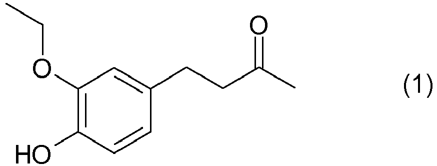 4-(3-éthoxy-4-hydroxyphényl)butan-2-one (éthyl gingérone) 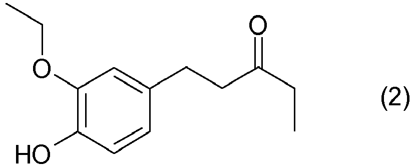 4-(3-éthoxy-4-hydroxyphényl)pentan-3-one 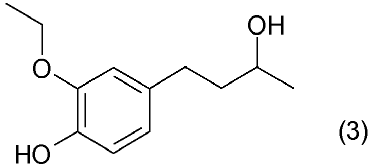 2-éthoxy-4-(3-hydroxybutyl)phénol

Selon une forme particulièrement préférée, on utilisera le composé (1).

Les composés de formule (I) peuvent être préparés aisément par l'homme du métier sur la base de ses connaissances générales. On peut notamment citer les références bibliographiques suivantes : J. Asian Natural Products Research, 2006, 8(8), 683-688; Helv. Chimica Acta, 2006, 89(3), 483-495; Chem. Pharm. Bull., 2006, 54(3), 377-379; et Bioorg. Med. Chem. Lett., 2004, 14(5), 1287-1289.

Ils peuvent ainsi être préparés à partir d'éthylvanilline de la manière suivante :

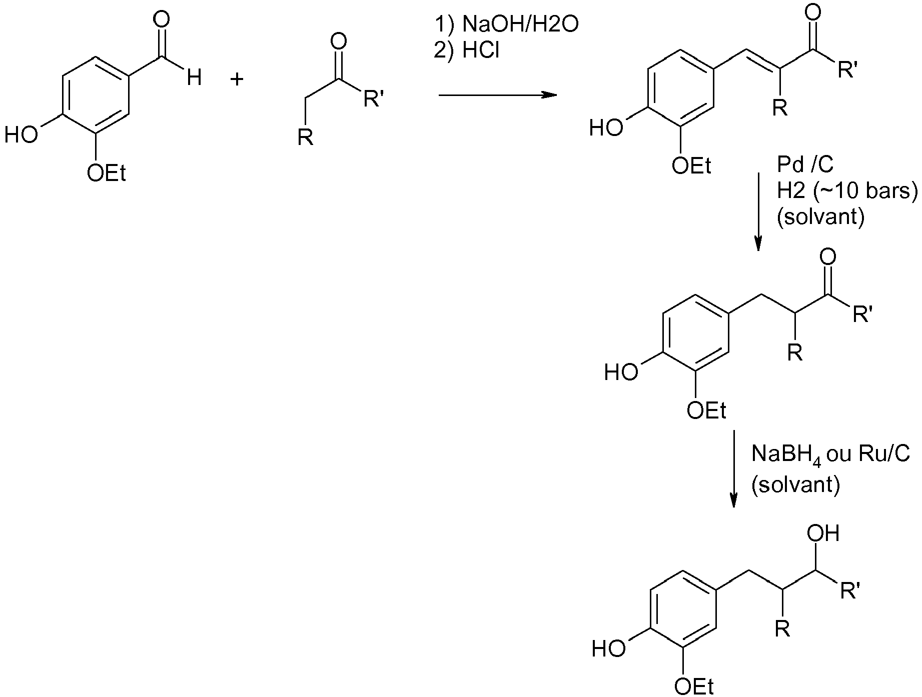

Les composés de formule (I) avec C-X représentant CHOH peuvent être obtenus par réduction des composés correspondants dans lesquels C-X représentent C=O, par exemple réduction avec Ru/C ou NaBH₄.

Le ou les composés de formule (I) conformes à l'invention sont présents dans la composition de préférence à des concentrations allant de 0,01 à 10% en poids et encore plus préférentiellement de 0,5 à 5% et encore plus particulièrement de 1 à 3% par rapport au poids total de la composition.

### FORMES GALENIQUES

La composition selon l'invention peut se présenter sous toutes les formes galéniques classiquement utilisées pour une application topique et notamment sous forme de gels aqueux, de solutions aqueuses ou hydroalcooliques. Elles peut aussi, par ajout d'une phase grasse ou huileuse, se présenter sous forme de dispersions du type lotion, d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-solide ou solide du type crème ou gel, ou encore d'émulsions multiples (E/H/E ou H/E/H), de microémulsions, de dispersions vésiculaires de type ionique et/ou non ionique, ou des dispersions cire/phase aqueuse. Ces compositions sont préparées selon les méthodes usuelles.

Les compositions peuvent être notamment conditionnées sous forme pressurisée dans un dispositif aérosol ou dans un flacon pompe ; conditionnée dans un dispositif muni d'une paroi ajourée notamment une grille ; conditionnées dans un dispositif muni d'un applicateur à billes ("roll-on) ; conditionnées sous forme de bâtonnets (sticks), sous forme de poudre libre ou compactée. Elles contiennent à cet égard les ingrédients généralement utilisés dans ce type de produits et bien connus de l'homme de l'art.

Selon une autre forme particulière de l'invention, les compositions selon l'invention peuvent être anhydres.

On entend par composition anhydre une composition contenant moins de 2 % en poids d'eau, voire moins de 0,5 % d'eau, et notamment exempte d'eau, l'eau n'étant pas ajoutée lors de la préparation de la composition mais correspondant à l'eau résiduelle apportée par les ingrédients mélangés.

Selon une autre forme particulière de l'invention, les compositions selon l'invention peuvent être solides en particulier sous forme de bâtonnet ou stick.

Par «composition solide», on entend que la mesure de la force maximale mesurée en texturométrie lors de l'enfoncement d'une sonde dans l'échantillon de formule doit être au moins égale à 0,25 Newton, en particulier au moins égal à 0,30 Newton, notamment au moins égale 0,35 Newton, appréciée dans des conditions de mesure précises comme suit.

Les formules sont coulées à chaud dans des pots de 4 cm de diamètre et 3 cm de fond. Le refroidissement est fait à température ambiante. La dureté des formules réalisées est mesurée après 24 heures d'attente. Les pots contenant les échantillons sont caractérisés en texturométrie à l'aide d'un texturomètre tel que celui commercialisé par la société Rhéo TA-XT2, selon le protocole suivant : une sonde de type bille en inox de diamètre 5 mm est amenée au contact de l'échantillon à une vitesse de 1 mm/s. Le système de mesure détecte l'interface avec l'échantillon avec un seuil de détection égal à 0,005 newtons. La sonde s'enfonce de 0,3 mm dans l'échantillon, à une vitesse de 0,1 mm/s. L'appareil de mesure enregistre l'évolution de la force mesurée en compression au cours du temps, pendant la phase de pénétration. La dureté de l'échantillon correspond à la moyenne des valeurs maximales de la force détectée pendant la pénétration, sur au moins 3 mesures.

### PHASE AQUEUSE

Les compositions selon l'invention destinées à l'usage cosmétique peuvent comporter au moins une phase aqueuse. Elles sont notamment formulées en lotions aqueuses ou en émulsion eau-dans-huile, huile-dans-eau, ou en émulsion multiple (émulsion triple huile-dans-eau-dans-huile ou eau-dans-huile-dans-eau (de telles émulsions sont connues et décrites par exemple par C. FOX dans « Cosmetics and Toiletries » - november 1986 - Vol 101 - pages 101-112).

La phase aqueuse des dites compositions contient de l'eau et en général d'autres solvants solubles ou miscibles dans l'eau. Les solvants solubles ou miscibles dans l'eau comprennent les mono alcools à chaîne courte par exemple en C₁-C₄ comme l'éthanol, l'isopropanol ; les diols ou les polyols comme l'éthylèneglycol, le 1,2-propylèneglycol, le 1,3-butylène glycol, l'hexylèneglycol, le diéthylèneglycol, le dipropylene glycol, le 2-éthoxyéthanol, le diéthylène glycol monométhyléther, le triéthylène glycol monométhyléther et le sorbitol. On utilisera plus particulièrement le propylèneglycol et la glycérine, le propane 1,3 diol.

### EMULSIONNANTS

### a) Emulsionnants huile-dans-eau

Comme émulsionnants pouvant être utilisés dans les émulsions huile-dans-eau ou émulsions triples huile-dans-eau-dans-huile, on peut citer par exemple les émulsionnants non ioniques tels que les esters d'acides gras et de glycérol oxyalkylénés (plus particulièrement polyoxyéthylénés) ; les esters d'acides gras et de sorbitan oxyalkylénés ; les esters d'acides gras oxyalkylénés (oxyéthylénés et/ou oxypropylénés) ; les éthers d'alcools gras oxyalkylénés (oxyéthylénés et/ou oxypropylénés) ; les esters de sucres comme le stéarate de sucrose ; et leurs mélanges tels que le mélange de stéarate de glycéryle et de stéarate de PEG-40.

On peut citer également les mélanges émulsionnants alcool gras/alkylpolyglycoside tels que sont décrits dans les demandes WO92/06778, WO95/13863 et WO98/47610 comme les produits commerciaux vendus par la société SEPPIC sous les appellation MONTANOV ®.

### b) Emulsionnants eau-dans-huile

Parmi les émulsionnants pouvant être utilisés dans les émulsions eau-dans-huile ou émulsions triples eau-dans-huile-dans-eau-dans-huile ou émulsions triples, on peut citer à titre d'exemple les alkyl dimethicone copolyols répondant à la formule (I) suivante 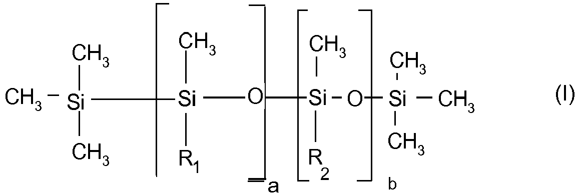 dans lesquelles :
R₁ désigne un groupement alkyle linéaire ou ramifié en C₁₂-C₂₀ et de préférence en C₁₂-C₁₈ ;
R₂ désigne le groupement :--CₙH₂ₙ--(-OC₂H₄-)ₓ--(-OC₃H₆-)_{y}--O-R₃,
R₃ désigne un atome d'hydrogène ou un radical akyle linéaire ou ramifié comportant de 1 à 12 atomes de carbone ;
a est un nombre entier allant de 1 à environ 500 ;
b désigne un nombre entier allant de 1 à environ 500 ;
n est un nombre entier allant de 2 à 12 et de préférence 2 à 5 ;
x désigne un nombre entier allant de 1 à environ 50 et de préférence de 1 à 30 ;
y désigne un nombre entier allant de 0 à environ 49 et de préférence 0 à 29 sous réserve que lorsque y est différent de zéro le ratio x/y est supérieur à 1 et de préférence varie de 2 à 11.

Parmi les émulsionnants alkyldimethicone copolyols de formule (I) préférés, on citera plus particulièrement le CETYL PEG/PPG-10/1 DIMETHICONE et plus particulièrement le mélange CETYL PEG/PPG-10/1 DIMETICONE AND DIMETHICONE (nom INCI) comme le produit vendu sous le nom commercial ABIL EM90 par la société GOLDSCHMIDT ou bien le mélange (POLYGLYCERYL-4-STEARATE and CETYL PEG/PPG-10 (AND) DIMETHICONE (AND) HEXYL LAURATE) comme le produit vendu sous le nom commercial ABIL WE09 par la même société.

Parmi les émulsionnants eau-dans-huile, on peut citer également les dimethicone copolyols répondant à la formule (II) suivante 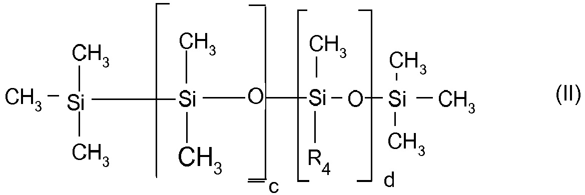 dans lesquelles
R₄ désigne le groupement :--CₘH₂ₘ--(-OC₂H₄-)ₛ--(-OC₃H₆-)ₜ--O-R₅,
R₅ désigne un atome d'hydrogène ou un radical akyle linéaire ou ramifié comportant de 1 à 12 atomes de carbone ;
c est un nombre entier allant de 1 à environ 500
d désigne un nombre entier allant de 1 à environ 500,
m est un nombre entier allant de 2 à 12 et de préférence 2 à 5 ,
s désigne un nombre entier allant de 1 à environ 50, et de préférence de 1 à 30 ;
t désigne un nombre entier allant de 0 à environ 50 et de préférence de 0 à 30 ; sous réserve que la somme s+t soit supérieure ou égal à 1.

Parmi ces émulsionnants dimethicone copolyols de formule (II) préférentiels on utilisera particulièrement le PEG-18/PPG-18 DIMETHICONE et plus particulièrement le mélange CYCLOPENTASILOXANE (and) PEG-18/PPG-18 DIMETHICONE (nom INCI) tel que le produit vendu par la société Dow Corning sous la dénomination commerciale Silicone DC 5225 C ou KF-6040 de la société Shin Etsu.

Selon une forme particulièrement préféré, on utilisera un mélange d'au moins un émulsionnant de formule (I) et d'au moins un émulsionnant de formule (II).

On utilisera plus particulièrement un mélange de PEG-18/PPG-18 Dimethicone et Cetyl PEG/PPG-10/1 DIMETHICONE et encore plus particulièrement un mélange de (CYCLOPENTASILOXANE (and) PEG-18/PPG-18 Dimethicone) et de Cetyl PEG/PPG-10/1 DIMETICONE and Dimethicone ou de (Polyglyceryl-4-stearate and Cetyl PEG/PPG-10 (and) Dimethicone (and) Hexyl Laurate).

Parmi les émulsionnants eau-dans-huile, on peut citer également les émulsionnants non ioniques dérivés d'acide gras et de polyol, les alkylpolyglycosides (APG), les esters de sucres et leurs mélanges.

Comme émulsionnants non ioniques dérivés d'acide gras et de polyol, on peut utiliser notamment les esters d'acide gras et de polyol, l'acide gras ayant notamment une chaîne alkyle en C8-C24, et les polyols étant par exemple le glycérol et le sorbitan.

Comme esters d'acide gras et de polyol, on peut citer notamment les esters d'acide isostéarique et de polyols, les esters d'acide stéarique et de polyols, et leurs mélanges, en particulier les esters d'acide isostéarique et de glycérol et/ou de sorbitan.

Comme esters d'acide stéarique et de polyols, on peut citer notamment les esters de polyéthylèneglycol comme le PEG-30 Dipolyhydroxystearate tel que le produit commercialisé sous le nom Arlacel P135 par la société ICI.

Comme esters de glycérol et/ou de sorbitan, on peut citer par exemple l'isostéarate de polyglycérol, tel que le produit commercialisé sous la dénomination Isolan GI 34 par la société Goldschmidt ; l'isostéarate de sorbitan, tel que le produit commercialisé sous la dénomination Arlacel 987 par la société ICI ; l'isostéarate de sorbitan et le glycérol, tel que le produit commercialisé sous la dénomination Arlacel 986 par la société ICI, , le mélange d'isostéarate de sorbitan et d'isostéarate de polyglycérol (3 moles) commercialisé sous la dénomination Arlacel 1690 par la société Unigema. et leurs mélanges.

L'émulsionnant peut être choisi aussi parmi les alkylpolyglycosides ayant un HLB inférieur à 7, par exemple ceux représentés par la formule générale (1) suivante :

R-O-(G)x (1)

dans laquelle R représente un radical alkyle ramifié et/ou insaturé, comportant de 14 à 24 atomes de carbone, G représente un sucre réduit comportant de 5 à 6 atomes de carbone, et x désigne une valeur allant de 1 à 10 et de préférence de 1 à 4, et G désigne notamment le glucose, le fructose ou le galactose.

Le radical alkyle insaturé peut comprendre une ou plusieurs insaturations éthyléniques, et en particulier une ou deux insaturations éthyléniques.

Comme alkylpolyglycosides de ce type, on peut citer les alkylpolyglucosides (G=glucose dans la formule (I)), et notamment les composés de formule (I) dans laquelle R représente plus particulièrement un radical oléyle (radical insaturé en C18) ou isostéaryle (radical saturé en C18), G désigne le glucose, x est une valeur allant de 1 à 2, notamment l'isostéaryl-glucoside, l'oléyl-glucoside et leurs mélanges. Cet alkylpolyglucoside peut être utilisé en mélange avec un co-émulsionnant, plus spécialement avec un alcool gras et notamment un alcool gras ayant la même chaîne grasse que celle de l'alkylpolyglucoside, c'est-à-dire comportant de 14 à 24 atomes de carbone et ayant une chaîne ramifiée et/ou insaturée, et par exemple l'alcool isostéarylique quand l'alkylpolyglucoside est l'isostéaryl-glucoside, et l'alcool oléylique quand l'alkylpolyglucoside est l'oleyl-glucoside, éventuellement sous forme d'une composition autoémulsionnante, comme décrit par exemple dans le document WO-A-92/06778. On peut utiliser par exemple le mélange d'isostéaryl-glucoside et d'alcool isostéarylique, commercialisé sous la dénomination Montanov WO 18 par la société SEPPIC ainsi que le mélange octyldodécanol et octyldodecylxyloside commercialisé sous la dénomination FLUDANOV 20X par la société SEPPIC.

On peut également citer les polyoléfines à terminaison succinique, comme les polyisobutylènes à terminaison succinique estérifiée et leurs sels, notamment les sels de diéthanolamine, tels que les produits commercialisés sous les dénominations Lubrizol 2724, Lubrizol 2722 et Lubrizol 5603 par la société Lubrizol ou le produit commercial CHEMCINNATE 2000.

La quantité totale en émulsionnants dans la composition sera de préférence dans la composition selon l'invention à des teneurs en matière active allant de 1 à 8% en poids et plus particulièrement de 2 à 6% en poids par rapport au poids total de la composition.

### PHASE GRASSE

Les compositions selon l'invention peuvent contenir au moins une phase liquide organique non-miscible dans l'eau appelée phase grasse. Celle-ci comprend en général un ou plusieurs composés hydrophobes qui rendent ladite phase non-miscible dans l'eau. Ladite phase est liquide (en l'absence d'agent structurant) à température ambiante (20-25 °C). De manière préférentielle, la phase organique liquide organique non-miscible dans l'eau conforme à l'invention est généralement constituée comprend généralement au moins une huile volatile et/ou une huile non volatile et éventuellement au moins un agent structurant.

Par « huile », on entend un corps gras liquide à température ambiante (25 °C) et pression atmosphérique (760mm de Hg soit 105 Pa). L'huile peut être volatile ou non volatile.

Par « huile volatile », on entend au sens de l'invention une huile susceptible de s'évaporer au contact de la peau ou de la fibre kératinique en moins d'une heure, à température ambiante et pression atmosphérique. Les huiles volatiles de l'invention sont des huiles cosmétiques volatiles, liquides à température ambiante, ayant une pression de vapeur non nulle, à température ambiante et pression atmosphérique, allant en particulier de 0,13 Pa à 40 000 Pa (10⁻³ à 300 mm de Hg), en particulier allant de 1,3 Pa à 13 000 Pa (0,01 à 100 mm de Hg), et plus particulièrement allant de 1,3 Pa à 1300 Pa (0,01 à 10 mm de Hg).

Par « huile non volatile », on entend une huile restant sur la peau ou la fibre kératinique à température ambiante et pression atmosphérique au moins plusieurs heures et ayant notamment une pression de vapeur inférieure à 10⁻³ mm de Hg (0,13 Pa).

L'huile peut être choisie parmi toutes les huiles physiologiquement acceptables et en particulier cosmétiquement acceptables, notamment les huiles minérales, animales, végétales, synthétiques ; en particulier les huiles hydrocarbonées et/ou siliconées et/ou fluorées volatiles ou non volatiles et leurs mélanges.

Plus précisément, par « huile hydrocarbonée », on entend une huile comportant principalement des atomes de carbone et d'hydrogène et éventuellement une ou plusieurs fonctions choisies parmi les fonctions hydroxyle, ester, éther, carboxylique. Généralement, l'huile présente une viscosité de 0,5 à 100 000 mPa.s, de préférence de 50 à 50 000 mPa.s et de préférence encore de 100 à 300 000 mPa.s.

A titre d'exemple d'huile volatile utilisable dans l'invention, on peut citer :
- les huiles hydrocarbonées volatiles choisies parmi les huiles hydrocarbonées ayant de 8 à 16 atomes de carbones, et notamment les isoalcanes en C₈-C₁₆ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars ou de Permetyls, les esters ramifiés en C₈-C₁₆, le néopentanoate d'iso-hexyle, et leurs mélanges. D'autres huiles hydrocarbonées volatiles comme les distillats de pétrole, notamment ceux vendus sous la dénomination Shell Soit par la société SHELL, peuvent aussi être utilisées ; les alcanes linéaires volatils comme ceux décrits dans la demande de brevet de la société Cognis DE10 2008 012 457.
- les silicones volatiles, comme par exemple les huiles de silicones linéaires ou cycliques volatiles, notamment celles ayant une viscosité ≤ 8 centistokes (8 10⁻⁶ m²/s), et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane
- et leurs mélanges.

On peut également citer les huiles linéaires alkyltrisiloxanes volatiles de formule générale (I) : 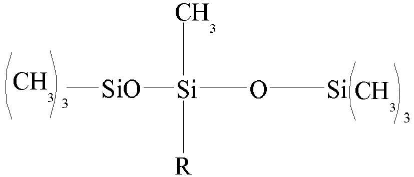 où R représente un groupe alkyle comprenant de 2 à 4 atomes de carbone et dont un ou plusieurs atomes d'hydrogène peuvent être substitués par un atome de fluor ou de chlore.

Parmi les huiles de formule générale (I), on peut citer :
le 3-butyl 1,1,1,3,5,5,5-heptaméthyl trisiloxane,
le 3-propyl 1,1,1,3,5,5,5-heptaméthyl trisiloxane, et
le 3-éthyl 1,1,1,3,5,5,5-heptaméthyl trisiloxane,
correspondant aux huiles de formule (I) pour lesquelles R est respectivement un groupe butyle, un groupe propyle ou un groupe éthyle.

A titre d'exemple d'huile non volatile utilisable dans l'invention, on peut citer :
- les huiles hydrocarbonées d'origine animale telles que le perhydrosqualène ;
- les huiles hydrocarbonées végétales telles que les triglycérides liquides d'acides gras de 4 à 24 atomes de carbone comme les triglycérides des acides heptanoïque ou octanoïque ou encore les huiles les huiles de germe de blé, d'olive, l'huile d'amande douce, de palme, de colza, de coton, de luzerne, de pavot, de potimarron, de courge, de cassis, d'onagre, de millet, d'orge, de quinoa, de seigle, de carthame, de bancoulier, de passiflore, de rosier muscat, de tournesol, de maïs, de soja, de courge, de pépins de raisin, de sésame, de noisette, d'abricot, de macadamia, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, de beurre de karité ;
- les hydrocarbures linéaires ou ramifiés, d'origine minérale ou synthétique tels que les huiles de paraffine et leurs dérivés, la vaseline, les polydécènes, les polybutènes, le polyisobutène hydrogéné tel que le Parleam, le squalane ;
- les éthers de synthèse ayant de 10 à 40 atomes de carbone ;
- les esters de synthèse notamment d'acides gras comme les huiles de formule R₁COOR₂ dans laquelle R₁ représente le reste d'un acide gras supérieur linéaire ou ramifié comportant de 1 à 40 atomes de carbone et R₂ représente une chaîne hydrocarbonée notamment ramifiée contenant de 1 à 40 atomes de carbone avec R₁ + R₂ ≥ 10 comme par exemple l'huile de Purcellin (octanoate de cétostéaryle), l'isononanoate d'isononyle, le myristate d'isopropyle, le palmitate d'isopropyle, le benzoate d'alcool en C₁₂ à C₁₅, le laurate d'hexyle, l'adipate de diisopropyle, l'isononanoate d'isononyle, le palmitate d'éthyl 2-hexyle, le stéarate d'octyl 2-dodécyle, l'érucate d'octyl 2-dodécyle, l'isostéarate d'isostéaryle, le tridecyl trimellitate ; les octanoates, décanoates ou ricinoléates d'alcools ou de polyalcools comme le dioctanoate de propylène glycol ; les esters hydroxylés comme l'isostéaryl lactate, l'octyl hydroxy stéarate, l'hydroxy stéarate d'octyl dodécyle, le diisostéaryl malate, le citrate de triisocétyle, des heptanoates, octanoates, décanoates d'alcools gras ; des esters de polyol comme le dioctanoate de propylène glycol, le diheptanoate de néopentyl glycol, le diisononanoate de diéthylène glycol ; et les esters du pentaérythritol comme le tétra-isostéarate de pentaérythrytyle ;
- des alcools gras liquides à température ambiante à chaîne carbonée ramifiée et/ou insaturée ayant de 12 à 26 atomes de carbone comme l'octyl dodécanol, l'alcool isostéarylique, le 2-butyloctanol, le 2-hexyl décanol, le 2-undécyl pentadécanol, l'alcool oléique ;
- les acides gras supérieurs tels que l'acide oléique, l'acide linoléique, l'acide linolénique ;
- les carbonates ;
- les acétates ;
- les citrates ;
- les huiles fluorées éventuellement partiellement hydrocarbonées et/ou siliconées comme les huiles fluorosiliconées, les polyéthers fluorés, les silicones fluorées telles que décrit dans le document EP-A-847752;
- les huiles siliconées comme les polydiméthylsiloxanes (PDMS) non volatiles, linéaires ou cycliques ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényl triméthicones, les phényl diméthicones, les phényl triméthylsiloxy diphényl siloxanes, les diphényl diméthicones, les diphényl méthyldiphényl trisiloxanes, les 2-phényl éthyl triméthyl-siloxysilicates, et
- leurs mélanges.

### AGENT STRUCTURANT

Les compositions selon l'invention comprenant une phase grasse peuvent contenir en plus au moins un agent structurant de ladite phase grasse qui peut être choisi de préférence parmi les cires, les composés pâteux, les gélifiants lipophiles minéraux ou organiques et leurs mélanges.

Il est entendu que la quantité en ces composés peut être ajustée par l'homme du métier de manière à ne pas porter préjudice à l'effet recherché dans le cadre de la présente invention.

### Cire(s)

La cire est d'une manière générale un composé lipophile, solide à température ambiante (25 °C), à changement d'état solide/liquide réversible, ayant un point de fusion supérieur ou égal à 30 °C pouvant aller jusqu'à 200 °C et notamment jusqu'à 120 °C.

En particulier, les cires convenant à l'invention peuvent présenter un point de fusion supérieur ou égal à 45 °C, et en particulier supérieur ou égal à 55 °C.

Au sens de l'invention, la température de fusion correspond à la température du pic le plus endothermique observé en analyse thermique (DSC) telle que décrite dans la norme ISO 11357-3 ; 1999. Le point de fusion de la cire peut être mesuré à l'aide d'un calorimètre à balayage différentiel (DSC), par exemple le calorimètre vendu sous la dénomination « MDSC 2920 » par la société TA Instruments.

### Le protocole de mesure est le suivant :

Un échantillon de 5 mg de cire disposé dans un creuset est soumis à une première montée en température allant de -20 °C à 100 °C, à la vitesse de chauffe de 10 °C/minute, puis est refroidi de 100 °C à -20 °C à une vitesse de refroidissement de 10 °C/minute et enfin soumis à une deuxième montée en température allant de -20 °C à 100 °C à une vitesse de chauffe de 5 °C/minute. Pendant la deuxième montée en température, on mesure la variation de la différence de puissance absorbée par le creuset vide et par le creuset contenant l'échantillon de cire en fonction de la température. Le point de fusion du composé est la valeur de la température correspondant au sommet du pic de la courbe représentant la variation de la différence de puissance absorbée en fonction de la température.

Les cires susceptibles d'être utilisées dans les compositions selon l'invention sont choisies parmi les cires, solides, à température ambiante d'origine animale, végétale, minérale ou de synthèse et leurs mélanges.

A titre illustratif des cires convenant à l'invention, on peut notamment citer les cires hydrocarbonées comme la cire d'abeille, la cire de lanoline, et les cires d'insectes de Chine, la cire de son de riz, la cire de Carnauba, la cire de Candellila, la cire d'Ouricury, la cire d'Alfa, la cire de berry, la cire de shellac, la cire du Japon et la cire de sumac; la cire de montan, les cires d'orange et de citron, Cire de Tournesol raffinée commercialisée sous la dénomination SUNFLOWER WAX par KOSTER KEUNEN, les cires microcristallines, les paraffines et l'ozokérite; les cires de polyéthylène, les cires obtenues par la synthèse de Fisher-Tropsch et les copolymères cireux ainsi que leurs esters.

On peut aussi citer des cires obtenues par hydrogénation catalytique d'huiles animales ou végétales ayant des chaînes grasses, linéaires ou ramifiées, en C₈-C₃₂. Parmi celles-ci, on peut notamment citer l'huile de jojoba isomérisée telle que l'huile de jojoba partiellement hydrogénée isomérisée trans fabriquée ou commercialisée par la société Desert Whale sous la référence commerciale Iso-Jojoba-50®, l'huile de tournesol hydrogénée, l'huile de ricin hydrogénée, l'huile de coprah hydrogénée, l'huile de lanoline hydrogénée, et le tétrastéarate de di-(triméthylol-1,1,1 propane) vendu sous la dénomination de Hest 2T-4S® par la société HETERENE.

On peut encore citer les cires de silicone (C₃₀-₄₅ ALKYL DIMETHICONE), les cires fluorées.

On peut également utiliser les cires obtenues par hydrogénation d'huile de ricin estérifiée avec l'alcool cétylique vendues sous les dénominations de Phytowax ricin 16L64® et 22L73® par la société SOPHIM. De telles cires sont décrites dans la demande FR-A- 2792190.

Comme cire, on peut utiliser un (hydroxystéaryloxy)stéarate d'alkyle en C₂₀-C₄₀ (le groupe alkyle comprenant de 20 à 40 atomes de carbone), seul ou en mélange.

Une telle cire est notamment vendue sous les dénominations « Kester Wax K 82 P® », « Hydroxypolyester K 82 P® » et « Kester Wax K 80 P® » par la société KOSTER KEUNEN.

Comme micro-cires pouvant être utilisées dans les compositions selon l'invention, on peut citer notamment les micro cires de carnauba telles que celle commercialisée sous la dénomination de MicroCare 350® par la société MICRO POWDERS, les micro cires de cire synthétique telles que celle commercialisée sous la dénomination de MicroEase 114S® par la société MICRO POWDERS, les micro cires constituées d'un mélange de cire de carnauba et de cire de polyéthylène telles que celles commercialisées sous les dénominations de Micro Care 300® et 310® par la société MICRO POWDERS, les micro cires constituées d'un mélange de cire de carnauba et de cire synthétique telles que celle commercialisée sous la dénomination Micro Care 325® par la société MICRO POWDERS, les micro cires de polyéthylène telles que celles commercialisées sous les dénominations de Micropoly 200®, 220®, 220L® et 250S® par la société MICRO POWDERS, les produits commerciaux PERFOMALEN 400 POLYETHYLENE et PERFORMALENE 500-L POLYETHYLENE de NEW PHASE TECHNOLOGIES, le PERFORMALENE 655 POLYETHYLENE ou les cires de paraffine comme la cire ayant pour nom INCI , MICROCRISTALLINE WAX and SYNTHETIC WAX et vendue sous le nom commercial MICROLEASE par la Société SOCHIBO. ; les micro cires de polytétrafluoroéthylène telles que celles commercialisées sous les dénominations de Microslip 519® et 519 L® par la société MICRO POWDERS.

La composition selon l'invention comprendra de préférence une teneur en cire(s) allant de 3 % à 20% en poids par rapport au poids total de la composition, en particulier de 5 à 15%, plus particulièrement de 6 à 15%.

Selon une forme particulière de l'invention, dans le cadre des compositions solides anhydres sous forme de stick, on utilisera des micro-cires de polyéthylène sous forme de cristallites de facteur de forme au moins égal à 2 ayant un point de fusion allant de 70 à 110°C et de préférence 70 à 100°C, afin de réduire voire supprimer la présence de strates dans la composition solide,

Ces cristallites en aiguilles et notamment leurs dimensions peuvent être caractérisées visuellement selon la méthode suivante.

La cire est déposée sur une lame de microscope, laquelle est posée sur une platine chauffante. La lame et la cire sont chauffées à une température généralement au moins supérieure de 5 °C à celle du point de fusion de la cire ou du mélange de cire considéré(e). A la fin de la fonte, le liquide ainsi obtenu et la lame de microscope sont laissés refroidir pour se solidifier. L'observation des cristallites est réalisée à l'aide d'un microscope optique de type Leica DMLB100, avec un objectif sélectionné en fonction de la taille des objets à visualiser, et en lumière polarisée. Les dimensions des cristallites sont mesurées à l'aide d'un logiciel d'analyse d'images tel que ceux commercialisés par la société Microvision.

Les cires de polyéthylène cristallites conformes à l'invention possèdent de préférence une longueur moyenne allant de 5 à 10 µm. Par «longueur moyenne », on désigne la dimension donnée par la distribution granulométrique statistique à la moitié de la population, dite D50.

On utilisera plus particulièrement un mélange de cires PERFOMALEN 400 POLYETHYLENE et PERFORMALENE 500-L POLYETHYLENE de NEW PHASE TECHNOLOGIES

### Composés pâteux

Par « composé pâteux » au sens de la présente invention, on entend un composé gras lipophile à changement d'état solide/liquide réversible, présentant à l'état solide une organisation cristalline anisotrope, et comportant à la température de 23 °C une fraction liquide et une fraction solide.

Le composé pâteux est de préférence choisi parmi les composés synthétiques et les composés d'origine végétale. Un composé pâteux peut être obtenu par synthèse à partir de produits de départ d'origine végétale.

Le composé pâteux peut avantageusement choisi parmi :
- la lanoline et ses dérivés,
- les composés siliconés polymères ou non,
- les composés fluorés polymères ou non,
- les polymères vinyliques, notamment :
- les homopolymères d'oléfines,
- les copolymères d'oléfines,
- les homopolymères et copolymères de diènes hydrogénés,
- les oligomères linéaires ou ramifiés, homo ou copolymères de (méth)acrylates d'alkyles ayant de préférence un groupement alkyle en C₈-C₃₀,
- les oligomères homo et copolymères d'esters vinyliques ayant des groupements alkyles en C₈-C₃₀, et
- les oligomères homo et copolymères de vinyléthers ayant des groupements alkyles en C₈-C₃₀,
- les polyéthers liposolubles résultant de la polyéthérification entre un ou plusieurs diols en C₂-C₁₀₀, de préférence en C₂-C₅₀,
- les esters,
- leurs mélanges.

Parmi les esters, on préfère notamment :
- les esters d'un glycérol oligomère, notamment les esters de diglycérol, en particulier les condensats d'acide adipique et de glycérol, pour lesquels une partie des groupes hydroxyles des glycérols ont réagi avec un mélange d'acides gras tels que l'acide stéarique, l'acide caprique, l'acide stéarique et l'acide isostéarique et l'acide 12-hydroxystéarique, à l'image notamment de ceux commercialisé sous la marque Softisan 649 par la société Sasol,
- le propionate d'arachidyle commercialisé sous la marque Waxenol 801 par Alzo,
- les esters de phytostérol,
- les triglycérides d'acides gras et leurs dérivés,
- les esters de pentaérythritol,
- les polyesters non réticulés résultant de la polycondensation entre un acide dicarboxylique ou un polyacide carboxylique linéaire ou ramifié en C4-C50 et un diol ou un polyol en C₂-C₅₀,
- les esters aliphatiques d'ester résultant de l'estérification d'un ester d'acide hydroxycarboxylique aliphatique par un acide carboxylique aliphatique,
- les polyesters résultant de l'estérification, par un acide polycarboxylique, d'un ester d'acide hydroxy carboxylique aliphatique, ledit ester comprenant au moins deux groupes hydroxyle tels que les produits Risocast DA-H ®, et Risocast DA-L ®,
- les esters de dimère diol et dimère diacide, le cas échéant, estérifiés sur leur(s) fonction(s) alcool(s) ou acide(s) libre(s) par des radicaux acides ou alcools tels que le Plandool-G,
- leurs mélanges.

Parmi les composés pâteux d'origine végétale, on choisira de préférence un mélange de stérols de soja et de pentaérythritol oxyéthyléné (5OE) oxypropyléné (5 OP), commercialisé sous la référence Lanolide par la société VEVY.

### Gélifiants lipophiles

### Gélifiants minéraux

Comme gélifiant lipophile minéral, on peut citer les argiles éventuellement modifiées comme les hectorites modifiées par un chlorure d'ammonium en C10 à C22, comme l'hectorite modifiée par du chlorure de di-stéaryl di-méthyl ammonium telle que, par exemple, celle commercialisée sous la dénomination de Bentone 38V® par la société ELEMENTIS.

On peut également citer la silice pyrogénée éventuellement traitée hydrophobe en surface dont la taille des particules est inférieure à 1 µm. Il est en effet possible de modifier chimiquement la surface de la silice, par réaction chimique générant une diminution du nombre de groupes silanol présents à la surface de la silice. On peut notamment substituer des groupes silanol par des groupements hydrophobes : on obtient alors une silice hydrophobe. Les groupements hydrophobes peuvent être des groupements triméthylsiloxyle, qui sont notamment obtenus par traitement de silice pyrogénée en présence de l'hexaméthyldisilazane. Des silices ainsi traitées sont dénommées « Silica silylate » selon le CTFA (8ème édition, 2000). Elles sont par exemple commercialisées sous les références Aerosil R812® par la société DEGUSSA, CAB-O-SIL TS-530® par la société CABOT, des groupements diméthylsilyloxyle ou polydiméthylsiloxane, qui sont notamment obtenus par traitement de silice pyrogénée en présence de polydiméthylsiloxane ou du diméthyldichlorosilane. Des silices ainsi traitées sont dénommées « Silica diméthyl silylate » selon le CTFA (8ème édition, 2000). Elles sont par exemple commercialisées sous les références Aerosil R972®, et Aerosil R974® par la société DEGUSSA, CAB-O-SIL TS-610® et CAB-O-SIL TS-720® par la société CABOT.

La silice pyrogénée hydrophobe présente en particulier une taille de particules pouvant être nanométrique à micrométrique, par exemple allant d'environ de 5 à 200 nm.

### Gélifiants organiques

Les gélifiants lipophiles organiques polymériques sont par exemple les organopolysiloxanes élastomériques partiellement ou totalement réticulés, de structure tridimensionnelle, comme ceux commercialisés sous les dénominations de KSG6®, KSG16® et de KSG18® par la société SHIN-ETSU, de Trefil E-505C® et Trefil E-506C® par la société DOW-CORNING, de Gransil SR-CYC®, SR DMF10®, SR-DC556®, SR 5CYC gel®, SR DMF 10 gel® et de SR DC 556 gel® par la société GRANT INDUSTRIES, de SF 1204® et de JK 113® par la société GENERAL ELECTRIC ; l'éthylcellulose comme celle vendue sous la dénomination Ethocel® par la société DOW CHEMICAL ; les galactommananes comportant de un à six, et en particulier de deux à quatre, groupes hydroxyle par ose, substitués par une chaîne alkyle saturée ou non, comme la gomme de guar alkylée par des chaînes alkyle en C1 à C6, et en particulier en C1 à C3 et leurs mélanges. Les copolymères séquencés de type « dibloc », « tribloc » ou « radial » du type polystyrène/polyisoprène, polystyrène/polybutadiène tels que ceux commercialisés sous la dénomination Luvitol HSB® par la société BASF, du type polystyrène/copoly(éthylène-propylène) tels que ceux commercialisés sous la dénomination de Kraton® par la société SHELL CHEMICAL CO ou encore du type polystyrène/copoly(éthylène-butylène), les mélanges de copolymères tribloc et radial (en étoile) dans l'isododécane tels que ceux commercialisé par la société PENRECO sous la dénomination Versagel® comme par exemple le mélange de copolymère tribloc butylène/éthylène/styrène et de copolymère étoile éthylène/propylène/styrène dans l'isododécane (Versagel M 5960).

Comme gélifiant lipophile, on peut encore citer les polymères de masse moléculaire moyenne en poids inférieure à 100 000, comportant a) un squelette polymérique ayant des motifs de répétition hydrocarbonés pourvus d'au moins un hétéroatome, et éventuellement b) au moins une chaîne grasse pendante et/ou au moins une chaîne grasse terminale éventuellement fonctionnalisées, ayant de 6 à 120 atomes de carbone et étant liées à ces motifs hydrocarbonés, telles que décrites dans les demandes WO-A-02/056847, WO-A-02/47619 en particulier les résines de polyamides (notamment comprenant des groupes alkyles ayant de 12 à 22 atomes de carbone) telles que celles décrites dans US-A-5783657.

Parmi les gélifiants lipophiles pouvant être utilisés dans les compositions selon l'invention, on peut encore citer les esters de dextrine et d'acide gras, tels que les palmitates de dextrine, notamment tels que ceux commercialisés sous les dénominations Rheopearl TL® ou Rheopearl KL® par la société CHIBA FLOUR.

On peut également utiliser les polyamides siliconés du type polyorganosiloxane tels que ceux décrits dans les documents US-A-5,874,069, US-A-5,919,441, US-A-6,051,216 et US-A-5,981,680.

Ces polymères siliconés peuvent appartenir aux deux familles suivantes :
- des polyorganosiloxanes comportant au moins deux groupes capables d'établir des interactions hydrogène, ces deux groupes étant situés dans la chaîne du polymère, et/ou
- des polyorganosiloxanes comportant au moins deux groupes capables d'établir des interactions hydrogène, ces deux groupes étant situés sur des greffons ou ramifications.

### AGENTS ANTI-TRANSPIRANTS ADDITIONNELS

La composition l'invention telle que définie précédemment peut contenir en plus un ou plusieurs agents anti-transpirants additionnels en particulier des sels ou complexes d'aluminium et/ou de zirconium.

Les sels ou complexes anti-transpirants conformes à l'invention sont choisis généralement parmi les sels ou complexes d'aluminium et/ou de zirconium. Il sont de préférence choisis parmi les halohydrates de d'aluminium ; les halohydrates d'aluminium et de zirconium, les complexes d'hydroxychlorure de zirconium et d'hydroxychlorure d'aluminium avec ou sans un acide aminé tels que ceux décrits dans le brevet US-3792068.

Parmi les sels d'aluminium, on peut citer en particulier le chlorhydrate d'aluminium sous forme activée ou non activée, l'aluminium chlorohydrex, le complexe aluminium chlorohydrex polyéthylèneglycol, le complexe aluminium chlorohydrex propylèneglycol, l'aluminium dichlorohydrate, le complexe aluminium dichlorohydrex polyéthylèneglycol, le complexe aluminium dichlorohydrex propylèneglycol, l'aluminium sesquichlorohydrate, le complexe aluminium sesquichlorohydrex polyéthylèneglycol, le complexe aluminium sesquichlorohydrex propylèneglycol, le sulfate d'aluminium tamponné par le lactate de sodium et d'aluminium.

Parmi les sels d'aluminium et de zirconium, on peut citer en particulier l'aluminium zirconium octachlorohydrate, l'aluminium zirconium pentachlorohydrate, l'aluminium zirconium tetrachlorohydrate, l'aluminium zirconium trichlorohydrate.

Les complexes d'hydroxychlorure de zirconium et d'hydroxychlorure d'aluminium avec un acide aminé sont généralement connus sous l'appellation ZAG (lorsque l'acide aminé est la glycine). Parmi ces produits on peut citer les complexes aluminium zirconium octachlorohydrex glycine, aluminium zirconium pentachlorohydrex glycine, aluminium zirconium tetrachlorohydrex glycine et aluminium zirconium trichlorohydrex glycine.

Les sels ou complexes anti-transpirants peuvent être présents dans la composition selon l'invention à raison d'environ 0,5 à 25% en poids par rapport au poids total de la composition.

Il est également possible d'ajouter des absorbeurs d'humidité comme par exemple les perlites et de préférence les perlites expansées.

Les perlites utilisables selon l'invention sont généralement des aluminosilicates d'origine volcanique et ont comme composition
70,0-75,0% en poids de silice SiO₂
12,0-15,0% en poids d'oxyde d'aluminium oxyde Al₂O₃
3,0-5,0% d'oxyde de sodium Na₂O
3,0-5,0% d'oxyde de potassium K2O
0,5-2% d'oxyde de fer Fe₂O₃ →
0,2-0,7% d'oxyde de magnésium MgO
0,5-1,5% d'oxyde de calcium CaO
0,05 - 0,15% d'oxyde de titane TiO₂

La perlite est broyée, séchée puis calibrée dans une première étape. Le produit obtenu dit Perlite Ore est de couleur grise et de taille de l'ordre de 100 µm.

La Perlite Ore est ensuite expansée (1000°C/2 secondes) pour donner des particules plus ou moins blanches. Lorsque la température atteint 850-900 °C, l'eau emprisonnée dans la structure du matériau se vaporise et entraîne l'expansion du matériau par rapport à son volume d'origine. Les particules de perlite expansées conformes à l'invention peuvent être obtenues par le procédé d'expansion décrit dans le brevet US 5,002,698.

De préférence, les particules de perlite utilisées seront broyées ; elles sont dans ce cas dites Expanded Milled Perlite (EMP). Elles ont de préférence une taille de particule définie par un diamètre médian D50 allant de 0,5 à 50 µm et de préférence de 0,5 à 40 µm.

De préférence, les particules de perlite utilisées présentent une densité apparente non tassée à 25°C allant de 10 et 400 kg/m3 (Norme DIN 53468) et de préférence de 10 et 300 kg/m³.

De préférence, les particules de perlite expansée selon l'invention ont une capacité d'absorption d'eau mesurée au WET POINT allant de 200 à 1500% et de préférence de 250 à 800%.

Le Wet Point correspond à la quantité d'eau qu'il faut additionner à 1 g de particule pour obtenir une pâte homogène. Cette méthode dérive directement de celle de la prise d'huile appliquée aux solvants. Les mesures sont faites de la même manière par l'intermédiaire du Wet Point et du Flow Point ayant respectivement comme définition suivante :
WET POINT : masse exprimée en grammes pour 100g de produit correspondant à l'obtention d'une pâte homogène lors de l'addition d'un solvant à une poudre.
FLOW POINT : masse exprimée en grammes pour 100g de produit à partir de laquelle la quantité de solvant est supérieure à la capacité de la poudre à le retenir. Cela se traduit par l'obtention d'un mélange plus ou moins homogène s'écoulant sur la plaque de verre.

Le Wet Point et le Flow point sont mesurés selon le protocole suivant :

### Protocole de mesure de l'absorption d'eau.

### 1) Matériel utilisé

Plaque de verre (25 x 25 mm)
Spatule (manche en bois et partie métallique (15 x 2,7mm)
Pinceau à poils de soie
Balance

### 2) Mode Opératoire

On dépose la plaque de verre sur la balance et on pèse 1g de particules de perlite. On dépose le bécher contenant le solvant ainsi que la liquipipette de prélèvement sur la balance. On ajoute progressivement le solvant à la poudre en malaxant régulièrement l'ensemble (toutes les 3 à 4 gouttes) à l'aide de la spatule On note la masse de solvant nécessaire à l'obtention du Wet Point. On ajoute à nouveau le solvant et on note la masse permettant d'arriver au Flow Point. On effectuera la moyenne sur 3 essais.

On utilisera en particulier les particules de perlite expansée vendues sous les noms commerciaux OPTIMAT 1430 OR ou OPTIMAT 2550 par la société WORLD MINERALS.

### AGENTS DEODORANTS

Les agents déodorants peuvent être des agents bactériostatiques ou des agents bactéricides agissant sur les germes des odeurs axillaires, comme le 2,4,4'-trichloro-2'-hydroxydiphényléther (®Triclosan), le 2,4-dichloro-2'-hydroxydiphényléther, le 3',4',5'-trichlorosalicylanilide, la 1-(3',4'-dichlorophenyl)-3-(4'-chlorophenyl)urée (®Triclocarban) ou le 3,7,11-triméthyldodéca-2,5,10-triénol (®Farnesol) ; les sels d'ammonium quaternaires comme les sels de cetyltrimethylammonium, les sels de cétylpyridinium, le DPTA (acide 1,3-diaminopropanetétraacétique), le 1,2 decanediol (SYMCLARIOL de la société Symrise), - les dérivés de glycérine comme par exemple le Caprylic/Capric Glycerides (CAPMUL MCM de Abitec), le Caprylate ou caprate de Glycerol (DERMOSOFT GMCY et DERMOSOFT GMC respectivement de STRAETMANS), le Polyglyceryl-2 Caprate (DERMOSOFT DGMC de STRAETMANS) les dérivés de biguanide comme les sels de polyhexaméthylène biguanide. - la chlorhexidine et ses sels; 4-Phenyl-4,4-dimethyl-2butanol (SYMDEO MPP de Symrise).
Parmi les actifs déodorants conformes à l'invention, on peut aussi citer également - les sels de zinc comme le salicylate de zinc, le gluconate de zinc, le pidolate de zinc ; le sulfate de zinc, le chlorure de zinc, le lactate de zinc, le phénolsulfonate de zinc ; l'acide salicylique et ses dérivés tels que l'acide n-octanoyl-5-salicylique.

Les actifs déodorants peuvent être des absorbeurs d'odeur comme les ricinoléate de zinc, le bicarbonate de sodium ; les Zéolithes métalliques ou non, les cyclodextrines, l'alun.

Il peut s'agit également d'un agent chélatant tel que la DISSOLVINE GL-47-S de Akzo Nobel, EDTA ; DPTA.

Il peut s'agir également de polyol de type glycérine, propane 1,3 propane diol (ZEMEA PROPANEDIOL commercialisé par Dupont tate and lyle bio products).

Ou encore d'inhibiteur enzymatique tel que le triethyl citrate.

En cas d'incompatibilité ou pour les stabiliser, certains des agents mentionnés ci-dessus peuvent être incorporés dans des sphérules, notamment des vésicules ioniques ou non-ioniques et/ou des particules (capsules et/ou sphères).

Les agents déodorants peuvent être présents de préférence dans les compositions selon l'invention dans des concentrations pondérales allant 0,01 à 15% en poids par rapport au poids total de la composition.

### POUDRE ORGANIQUE

Selon une forme particulière de l'invention, les compositions selon l'invention contiendront en plus une poudre organique.

On entend dans la présente demande par « poudre organique », tout solide insoluble dans le milieu à température ambiante (25°C).

Comme poudres organiques qui peuvent être utilisées dans la composition de l'invention, on peut citer par exemple, les particules de polyamide et notamment celles vendues sous les dénominations ORGASOL par la société Atochem ; les fibres de nylon 6,6 notamment les fibres de polyamide commercialisées par les Etablissements P Bonte sous le nom Polyamide 0.9 Dtex 0.3 mm (non INCI : Nylon 6,6 ou Polyamide-6,6) ayant un diamètre moyen de 6 µm, un poids d'environ 0,9 dtex et une longueur allant de 0,3 mm à 1,5 mm ; les poudres de polyéthylène ; les microsphères à base de copolymères acryliques, telles que celles en copolymère diméthacrylate d'éthylène glycol/ methacrylate de lauryle vendues par la société Dow Corning sous la dénomination de POLYTRAP ; les microsphères de polyméthacrylate de méthyle, commercialisées sous la dénomination MICROSPHERE M-100 par la société Matsumoto ou sous la dénomination COVABEAD LH85 par la société Wackherr ; les microsphères de poly methacrylate de methyle creuses (granulometrie : 6,5 - 10,5 µ) commercialisées sous la dénomination GANZPEARL GMP 0800 par Ganz Chemical; micro-billes de copolymere methacrylate de methyle/dimethacrylate d'ethylene glycol (taille: 6.5-10.5 µ) commercialisées sous la dénomination GANZPEARL GMP 0820 par Ganz Chemical ou MICROSPONGE 5640 par la société Amcol Health & Beauty Solutions; les poudres de copolymère éthylène-acrylate, comme celles commercialisées sous la dénomination FLOBEADS par la société Sumitomo Seika Chemicals ; les poudres expansées telles que les microsphères creuses et notamment, les microsphères formées d'un terpolymère de chlorure de vinylidène, d'acrylonitrile et de méthacrylate et commercialisées sous la dénomination EXPANCEL par la société Kemanord Plast sous les références 551 DE 12 (granulométrie d'environ 12 µm et masse volumique 40 kg/m³), 551 DE 20 (granulométrie d'environ 30 µm et masse volumique 65 kg/m³), 551 DE 50 (granulométrie d'environ 40 µm), ou les microsphères commercialisées sous la dénomination MICROPEARL F 80 ED par la société Matsumoto ; les poudres de matériaux organiques naturels tels que les poudres d'amidon, notamment d'amidons de maïs, de blé ou de riz, réticulés ou non, telles que les poudres d'amidon réticulé par l'anhydride octénylsuccinate, commercialisées sous la dénomination DRY-FLO par la société National Starch ; les microbilles de résine de silicone telles que celles commercialisées sous la dénomination TOSPEARL par la société Toshiba Silicone, notamment TOSPEARL 240 ; les poudres d'aminoacides telles que la poudre de Lauroyllysine commercialisée sous la dénomination AMIHOPE LL-11 par la Société Ajinomoto ; les particules de microdispersion de cire, qui ont de préférence des dimensions moyennes inférieures à 1 µm et notamment allant de 0,02 µm à 1 µm, et qui sont constituées essentiellement d'une cire ou d'un mélange de cires, telles que les produits commercialisés sous la dénomination Aquacer par la société Byk Cera, et notamment : Aquacer 520 (mélange de cires synthétiques et naturelles), Aquacer 514 ou 513 (cire de polyéthylène), Aquacer 511 (cire polymérique), ou telles que les produits commercialisés sous la dénomination Jonwax 120 par la société Johnson Polymer (mélange de cires de polyéthylène et de paraffine) et sous la dénomination Ceraflour 961 par la société Byk Cera (cire de polyéthylène modifiée micronisée) ; et leurs mélanges.

### ADDITIFS

Les compositions cosmétiques selon l'invention peuvent comprendre en outre des adjuvants cosmétiques choisis parmi les adoucissants, les antioxydants, les opacifiants, les stabilisants, les agents hydratants, les vitamines, des bactéricides, les conservateurs, les polymères, les parfums, les agents épaississants ou de mise en suspension, des agents propulseurs ou tout autre ingrédient habituellement utilisé en cosmétique pour ce type d'application.

Bien entendu, l'homme de métier veillera à choisir ce ou ces éventuels composés complémentaires de manière telle que les propriétés avantageuses attachées intrinsèquement à la composition cosmétique conforme à l'invention ne soient pas, ou substantiellement pas, altérées par la ou les adjonctions envisagées.

Selon un mode de réalisation, une composition selon l'invention ne comprendra pas d'huile essentielle.

### EPAISSISSANTS ET AGENTS DE SUSPENSION

Les épaississants peuvent être choisis parmi les les polymères carboxyvinyliques tels que les Carbopols (Carbomers) et les Pemulen (Copolymère acrylate/C₁₀-C₃₀-alkylacrylate) ; les polyacrylamides comme par exemple les copolymères réticulés vendus sous les noms Sepigel 305 (nom C.T.F.A. : polyacrylamide/C₁₃-₁₄ isoparaffin/Laureth 7) ou Simulgel 600 (nom C.T.F.A. : acrylamide / sodium acryloyldimethyltaurate copolymer / isohexadecane / polysorbate 80) par la société Seppic ; les polymères et copolymères d'acide 2-acrylamido 2-méthylpropane sulfonique, éventuellement réticulés et/ou neutralisés, comme le poly(acide 2-acrylamido 2-méthylpropane sulfonique) commercialisé par la société Hoechst sous la dénomination commerciale « Hostacerin AMPS » (nom CTFA : ammonium polyacryloyldimethyl taurate ou le SIMULGEL 800 commercialisé par la société SEPPIC (nom CTFA : sodium polyacryolyldimethyl taurate / polysorbate 80 / sorbitan oleate) ; les copolymères d'acide 2-acrylamido 2-méthylpropane sulfonique et d'hydroxyethyl acrylate comme le SIMULGEL NS et le SEPINOV EMT 10 commercialisés par la société SEPPIC ; les dérivés cellulosiques tels que l'hydroxyéthylcellulose, la cétylhydroxyéthylcellulose ; les polysaccharides et notamment les gommes telles que la gomme de Xanthane, les gommes de guar hydroxypropylée ; les silices comme par exemple la Bentone Gel MIO vendue par la société NL INDUSTRIES ou la Veegum Ultra, vendue par la société POLYPLASTIC.

Les épaississants peuvent également être cationiques comme par exemple le POLYQUATERNIUM-37 commercialisé sous la dénomination Salcare SC95 (Polyquaternium-37 (And) Mineral Oil (And) PPG-1 Trideceth-6) ou Salcare SC96 (Polyquaternium-37 (And) Propylene Glycol Dicaprylate/Dicaprate (And) PPG-1-Trideceth-6) ou d'autre polymère cationiques réticulés comme par exemple ceux de nom CTFA Copolymère Ethylacrylate / Dimethylamino Ethyl Methacrylate Cationique En Emulsion.

### AGENTS DE SUSPENSION

Afin d'améliorer l'homogénéité du produit, on peut utiliser en plus un ou plusieurs agents de suspension qui sont choisis de préférence parmi les argiles montmorillonites modifiées hydrophobes comme les bentonites ou hectorites modifiées hydrophobes. On peut citer par exemple le produit Stearalkonium Bentonite (nom CTFA) (produit de réaction de la bentonite et de l'ammonium quaternaire chlorure de stéaralkonium) tel que le produit commercial vendu sous le nom TIXOGEL MP 250 par la société Sud Chemie Rheologicals, United Catalysts Inc ou le produit Disteardimonium Hectorite (nom CTFA) (produit de réaction de l'hectorite et du chlorure de distéaryldimonium) vendu sous le nom de Bentone 38 ou Bentone Gel par la société Elementis Specialities.

D'autres agents de suspension peuvent être utilisés, en l'occurrence dans des milieux hydrophiles (aqueux et/ou éthanoliques). Il s'agit de dérivés cellulosiques, de xanthane, guar amidon, caroube, agar agar.

Les agents de suspension sont présents de préférence dans des quantités allant de 0,1 à 5% en poids et plus préférentiellement de 0.2 à 2% en poids par rapport au poids total de la composition.

Les quantités de ces différents constituants pouvant être présents dans la composition cosmétique selon l'invention sont celles classiquement utilisées dans compositions pour le traitement de la transpiration.

### AEROSOLS

Les compositions selon l'invention peuvent encore être pressurisées et être conditionnées dans un dispositif aérosol constitué par :
(A) un récipient comprenant une composition anti-transpirante telle que définie précédemment,
(B) au moins un agent propulseur et un moyen de distribution de la dite composition aérosol.

Les propulseurs généralement utilisés dans ce type de produits et bien connus de l'homme de l'art, sont comme par exemple le diméthyléther (DME) ; les hydrocarbures volatils tels que le n-butane, le propane, l'isobutane, et leurs mélanges, éventuellement avec au moins un hydrocarbure chloré et/ou fluoré; parmi ces derniers on peut citer les composés vendus par la société Dupont de Nemours sous les dénominations Fréon® et Dymel®, et en particulier le monofluorotrichlorométhane, le difluorodichlorométhane, le tétrafluorodichloroéthane et le 1,1-difluoroéthane vendu notamment sous la dénomination commerciale DYMEL 152 A par la société DUPONT. On peut également utiliser en tant qu'agent propulseur le gaz carbonique, le protoxyde d'azote, l'azote ou l'air comprimé.

Les compositions contenant les particules de perlite telles que définies précédemment et le ou les agents propulseurs peuvent se trouver dans le même compartiment ou dans des compartiments différents dans le récipient aérosol. Selon l'invention, la concentration en agent propulseur varie généralement de 5 à 95% en poids pressurisée et plus préférentiellement de 50 à 85% en poids par rapport au poids total de la composition pressurisée.

Le moyen de distribution, qui forme une partie du dispositif aérosol, est généralement constitué par une valve de distribution commandée par une tête de distribution, elle même comprenant une buse par laquelle la composition aérosol est vaporisée. Le récipient contenant la composition pressurisée peut être opaque ou transparent. Il peut être en verre, en matériau polymérique ou en métal, recouvert éventuellement d'une couche de vernis protecteur.

### Exemple 1 : Stick savon :

| | |
|---|---|
| Ethyle gingerone | 2 g |
| Stéarate de sodium | 3,50 g |
| Acide béhénique (extrait de colza) | 0,75 g |
| Alcool stéarylique oxyéthyléné (100 OE) | 3,00 g |
| Acide éthylènediamine tétracétique (EDTA) | 0,50 g |
| Hydroxyde de sodium pur qsp pH=7 | |
| Glycérine | 20,00 g |
| Propyleneglycol | 50,00 g |
| Eau désionisée | qsp 100 |

La composition présente un effet déodorant.

### MISE EN EVIDENCE DE L'ACTIVITE BACTERICIDE D'UNE COMPOSITION VIS-A-VIS DES MICRO-ORGANISMES IMPLIQUES DANS LES ODEURS AXILLAIRES

Le test décrit ici permet la détermination quantitative de l'activité bactéricide d'une composition sur des germes en conditions optimales de croissance à savoir des germes du type Corynebacterium xerosis (Collection de l'Institut Pasteur n° 5216) cultivés sur gélose Tryptocaséine soja en pente.

La veille du test, dans un pilulier, on dépose 32 g de bouillon Tryptocaséine soja et l'on met à incuber à 35°C. Le jour du test, on ajoute 4 g de la composition à tester et on homogénéise au Vortex.

Un témoin de croissance sans produit est préparé dans les mêmes conditions (4g de diluant tryptone-sel à la place de la composition à tester) afin de vérifier que les germes sont dans des conditions de croissance favorables pendant toute la durée du test.

Pour la préparation de l'inoculum, cinq jours avant le début du test, on pratique un repiquage de la souche bactérienne sur milieu approprié. On incube 5 jours à 35°C. Le jour du test, on lave la pente avec environ 9 ml de diluant: La suspension obtenue titre à 10⁸ germes/ml (on effectue un dénombrement).

Le jour du test, on introduit 4 ml d'inoculum dans le pilulier renfermant la composition à tester ainsi que dans le pilulier témoin, ce qui correspond à un taux de 10⁷ bactéries par gramme de préparation. On place les piluliers dans un incubateur-agitateur (35°C - 200 RPM).

Après 24 heures de contact, on homogénéise le contenu des piluliers au Vortex. On effectue des dilutions décimales dans un milieu neutralisant (bouillon Eugon LT100). On dépose en surface de boîtes de Pétri gélosée (milieu Eugon LT 100). On incube les boîtes de Pétri 5 jours à l'étuve à 35°C. On procède au comptage des colonies sur les boîtes contenant plus de 20 et moins de 200 colonies.

On calcule la différence entre le logarithme décimal du nombre de bactéries retrouvé après 24 h de contact pour la composition testée et le logarithme décimal du nombre de bactéries retrouvé après 24 h de contact pour le témoin.

### On a réalisé l'exemple 2 suivant qui est un gel aqueux déodorant:

| | |
|---|---|
| ACRYLATES/C-10-C30 ALKYL ACRYLATE CROSSPOLYMER (CARBOPOL ULTREZ 20 POLYMER - LUBRIZOL) | 0,9 g |
| Hydroxyde de sodium pur qsp pH=7 | |
| Ethyle gingerone | 2 g |
| Polyethyleneglycol (8 OE) ou PEG-8 | 6 g |
| Acide Citrique | 0,33 g |
| Eau déminéralisée | qsp 100 g |

Les résultats d'activité déodorante obtenus sur la composition de l'exemple 2 sont résumés dans le tableau suivant :

| **Composition** | **Réduction après 24 heures de la population de microorganismes par rapport au témoin** |
|---|---|
| Souche | *Corynebacterium xerosis* |
| 2 (invention) | 1 log |

La composition 2 présente un effet déodorant.

## Revendications

1. Procédé cosmétique pour traiter les odeurs corporelles humaines en particulier les odeurs axillaires consistant à appliquer sur les matières kératiniques humaines une composition contenant dans un milieu cosmétiquement acceptable au moins un composé 4-(3-éthoxy-4-hydroxyphényl)alkylcétone ou 2-éthoxy 4-hydroxyalkylphénol de formule (I) suivante : 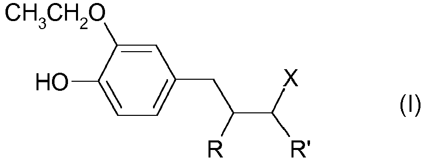 dans laquelle :
- R représente un atome d'hydrogène, ou un radical hydrocarboné, saturé ou insaturé (alkyle ou alcényle), linéaire ou ramifié, en C₁-C₆ ;
- R' représente un radical hydrocarboné, saturé ou insaturé (alkyle ou alcényle), linéaire ou ramifié, en C₁-C₁₈, éventuellement substitué par un groupe hydroxyle;
- C-X représente C=O ou CH-OH.

2. Procédé selon la revendication 1, où les composés de formule (I) sont choisis parmi ceux pour lesquels :
R représente H, méthyle ou éthyle.
R' représente un radical hydrocarboné linéaire, saturé en C₁-C₆ ou insaturé en C₂-C₆, éventuellement substitué par un groupe hydroxyle.

3. Procédé selon la revendication 1 ou 2, où les composés de formule (I) sont choisis parmi ceux pour lesquels :
- lorsque - C-X représente C=O, R désigne hydrogène et R' représente un radical alkyle linéaire en C₁-C₆, éventuellement substitué par un OH et plus préférentiellement R' désigne méthyle ou éthyle; ou bien
- lorsque - C-X représente CH-OH, R désigne hydrogène et R' représente un radical alkyle linéaire en C₁-C₆, éventuellement substitué par un OH et plus préférentiellement R' désigne méthyle ou éthyle.

4. Procédé selon l'une quelconque des revendications 1 à 3, où les composés de formule (I) sont choisis parmi les composés (1), (2) et (3) suivants :

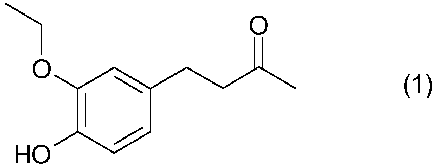

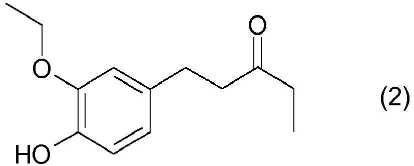

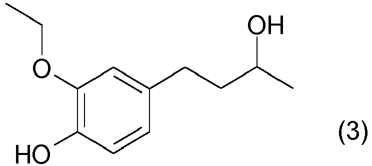

5. Procédé selon la revendication 4, où le composé de formule (I) est le composé (1) de structure :

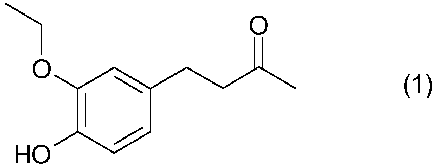

6. Procédé selon l'une quelconque des revendications 1 à 4, où le ou les composés de formule (I) sont présents à des concentrations allant de 0,01 à 10% en poids et encore plus préférentiellement de 0,5 à 5% et encore plus particulièrement de 1 à 3% par rapport au poids total de la composition.

7. Procédé selon l'une quelconque des revendications 1 à 6, où la composition ne comprend pas d'huile essentielle.

8. Procédé selon l'une quelconque des revendications 1 à 6, où la composition est différente d'une composition comprenant au moins une huile essentielle et de 0,5 à 5% en poids d'au moins un composé 4-(3-éthoxy-4-hydroxyphényl)alkylcétone de formule (I) où - C-X représente C=O.

9. Utilisation cosmétique d'au moins un composé de formule (I) selon l'une quelconque des revendications 1 à 5 comme actif déodorant.

10. Utilisation selon la revendication 9, où le composé de formule (I) est contenu dans une composition comprenant un milieu cosmétiquement acceptable

11. Utilisation selon la revendication 9 ou 10, où la composition ne comprend pas d'huile essentielle.

12. Utilisation selon la revendication 9 ou 10, où la composition est différente d'une composition comprenant au moins une huile essentielle et de 0,5 à 5% en poids d'au moins un composé 4-(3-éthoxy-4-hydroxyphényl)alkylcétone de formule (I) où - C-X représente C=O.

13. Composition conditionnée
(i) sous forme pressurisée dans un dispositif aérosol ou dans un flacon pompe ;
(ii) dans un dispositif muni d'une paroi ajourée notamment une grille ;
(iii) dans un dispositif muni d'un applicateur à billes ("roll-on) ;
(iv) sous forme de bâtonnet (sticks),
(v) sous forme de poudre libre ou compactée, **caractérisée par le fait qu'**elle contient dans un milieu cosmétiquement acceptable au moins un composé de formule (I) I tel que défini dans l'une quelconque des revendications 1 à 5 ;
ladite composition comportant en plus au moins un sel ou complexe anti-transpirant et/ou au moins un actif déodorant additionnel.

14. Composition selon la revendication 13, **caractérisée par le fait qu'**elle ne comprend pas d'huile essentielle.

15. Composition selon la revendication 13, **caractérisée par le fait qu'**elle est différente d'une composition comprenant au moins une huile essentielle et de 0,5 à 5% en poids d'au moins un composé 4-(3-éthoxy-4-hydroxyphényl)alkylcétone de formule (I) où - C-X représente C=O.

16. Composition, **caractérisée par le fait qu'**elle contient dans un milieu cosmétiquement acceptable au moins un composé de formule (I) tel que défini dans l'une quelconque des revendications 1 à 5 et au moins un sel ou complexe anti-transpirant et/ou au moins un actif déodorant additionnel.

17. Composition selon la revendication 16, **caractérisée par le fait qu'**elle ne comprend pas d'huile essentielle.

18. Composition selon la revendication 16, **caractérisée par le fait qu'**elle est différente d'une composition comprenant au moins une huile essentielle et de 0,5 à 5% en poids d'au moins un composé 4-(3-éthoxy-4-hydroxyphényl)alkylcétone de formule (I) où - C-X représente C=O.

## Patentansprüche

1. Kosmetisches Verfahren zur Behandlung von menschlichen Körpergerüchen, insbesondere Achselgerüchen, das darin besteht, dass man auf menschlichen Keratinmaterialien eine Zusammensetzung aufbringt, die in einem kosmetisch unbedenklichen Medium mindestens eine 4-(3-Ethoxy-4-hydroxyphenyl)alkylketon- oder 2-Ethoxy-4-hydroxyalkylphenol-Verbindung der nachstehenden Formel (I) enthält: 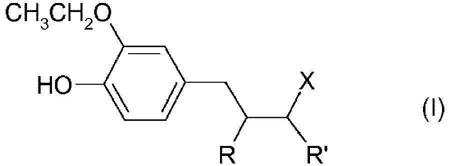 in der:
- R für ein Wasserstoffatom oder einen linearen oder verzweigten, gesättigten oder ungesättigten (Alkyl- oder Alkenyl-) C₁-C₆-Kohlenwasserstoffrest steht;
- R' für einen linearen oder verzweigten, gesättigten oder ungesättigten (Alkyl- oder Alkenyl-) C₁-C₁₈-Kohlenwasserstoffrest, der gegebenenfalls durch eine Hydroxylgruppe substituiert ist, steht;
- C-X für C=O oder CH-OH steht.

2. Verfahren nach Anspruch 1, wobei die Verbindungen der Formel (I) aus denjenigen ausgewählt werden, für die:
R für H, Methyl oder Ethyl steht,
R' für einen linearen, gesättigten C₁-C₆-Kohlenwasserstoffrest oder ungesättigten C₂-C₆-Kohlenwasserstoffrest, der gegebenenfalls durch eine Hydroxylgruppe substituiert ist, steht.

3. Verfahren nach Anspruch 1 oder 2, wobei die Verbindungen der Formel (I) aus denjenigen ausgewählt werden, für die:
- wenn C-X für C=O steht, R Wasserstoff bedeutet und R' für einen linearen C₁-C₆-Alkylrest, der gegebenenfalls durch ein OH substituiert ist, steht und weiter bevorzugt R' Methyl oder Ethyl bedeutet; oder auch
- wenn C-X für CH-OH steht, R Wasserstoff bedeutet und R' für einen linearen C₁-C₆-Alkylrest, der gegebenenfalls durch ein OH substituiert ist, steht und weiter bevorzugt R' Methyl oder Ethyl bedeutet.

4. Verfahren nach einem der Ansprüche 1 bis 3, bei dem die Verbindungen der Formel (I) aus den folgenden Verbindungen (1), (2) und (3) ausgewählt werden:

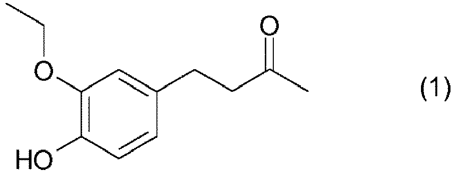

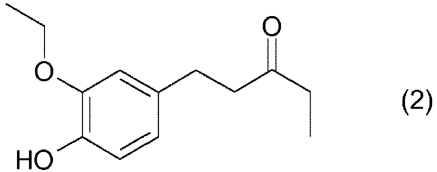

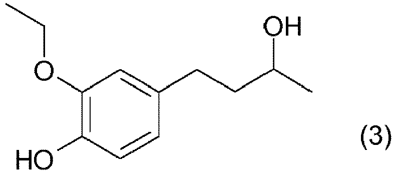

5. Verfahren nach Anspruch 4, wobei es sich bei der Verbindung der Formel (I) um die Verbindung (1) mit der folgenden Struktur handelt:

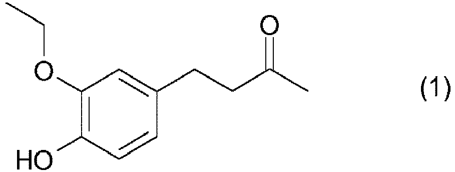

6. Verfahren nach einem der Ansprüche 1 bis 4, bei dem die Verbindung bzw. die Verbindungen der Formel (I) in Konzentrationen im Bereich von 0,01 bis 10 Gew.-% und noch weiter bevorzugt von 0,5 bis 5 Gew.-% und noch spezieller von 1 bis 3 Gew.-%, bezogen auf das Gesamtgewicht der Zusammensetzung, vorliegt bzw. vorliegen.

7. Verfahren nach einem der Ansprüche 1 bis 6, wobei die Zusammensetzung kein etherisches Öl umfasst.

8. Verfahren nach einem der Ansprüche 1 bis 6, wobei die Zusammensetzung von einer Zusammensetzung, die mindestens ein etherisches Öl und 0,5 bis 5 Gew.-% mindestens einer 4-(3-Ethoxy-4-hydroxyphenyl)-alkylketon-Verbindung der Formel (I), wobei -C-X für C=O steht, umfasst, verschieden ist.

9. Kosmetische Verwendung mindestens einer Verbindung der Formel (I) nach einem der Ansprüche 1 bis 5 als desodorierender Wirkstoff.

10. Verwendung nach Anspruch 9, wobei die Verbindung der Formel (I) in einer Zusammensetzung, die ein kosmetisch unbedenkliches Medium umfasst, enthalten ist.

11. Verwendung nach Anspruch 9 oder 10, wobei die Zusammensetzung kein etherisches Öl umfasst.

12. Verwendung nach Anspruch 9 oder 10, wobei die Zusammensetzung von einer Zusammensetzung, die mindestens ein etherisches Öl und 0,5 bis 5 Gew.-% mindestens einer 4-(3-Ethoxy-4-hydroxyphenyl)-alkylketon-Verbindung der Formel (I), wobei -C-X für C=O steht, umfasst, verschieden ist.

13. Zusammensetzung, die
(i) druckbeaufschlagt in einer Aerosolvorrichtung oder einer Pumpflasche;
(ii) in einer Vorrichtung mit einer durchbrochenen Wand, insbesondere einem Gitter;
(iii) in einer Vorrichtung mit einem Kugelapplikator ("Roller");
(iv) in Form eines Stifts;
(v) in Form eines losen oder kompaktierten Puders konfektioniert ist, **dadurch gekennzeichnet, dass** sie in einem kosmetisch unbedenklichen Medium mindestens eine Verbindung der Formel (I) gemäß einem der Ansprüche 1 bis 5 enthält;
wobei die Zusammensetzung außerdem mindestens ein schweißhemmend wirkendes Salz oder einen schweißhemmend wirkenden Komplex und/oder mindestens einen zusätzlichen desodorierenden Wirkstoff umfasst.

14. Zusammensetzung nach Anspruch 13, **dadurch gekennzeichnet, dass** sie kein etherisches Öl umfasst.

15. Zusammensetzung nach Anspruch 13, **dadurch gekennzeichnet, dass** sie von einer Zusammensetzung, die mindestens ein etherisches Öl und 0,5 bis 5 Gew.-% mindestens einer 4-(3-Ethoxy-4-hydroxyphenyl)-alkylketon-Verbindung der Formel (I), wobei -C-X für C=O steht, umfasst, verschieden ist.

16. Zusammensetzung, **dadurch gekennzeichnet, dass** sie in einem kosmetisch unbedenklichen Medium mindestens eine Verbindung der Formel (I) gemäß einem der Ansprüche 1 bis 5 und mindestens ein schweißhemmend wirkendes Salz oder einen schweißhemmend wirkenden Komplex und/oder mindestens einen zusätzlichen desodorierenden Wirkstoff enthält.

17. Zusammensetzung nach Anspruch 16, **dadurch gekennzeichnet, dass** sie kein etherisches Öl umfasst.

18. Zusammensetzung nach Anspruch 16, **dadurch gekennzeichnet, dass** sie von einer Zusammensetzung, die mindestens ein etherisches Öl und 0,5 bis 5 Gew.-% mindestens einer 4-(3-Ethoxy-4-hydroxyphenyl)-alkylketon-Verbindung der Formel (I), wobei -C-X für C=O steht, umfasst, verschieden ist.

## Claims

1. Cosmetic process for treating human body odor, in particular underarm odor, which consists in applying to human keratin materials a composition containing, in a cosmetically acceptable medium, at least one 4-(3-ethoxy-4-hydroxyphenyl)alkyl ketone or 2-ethoxy-4-hydroxyalkylphenol compound of formula (I) below: 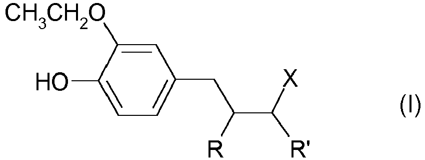 in which:
- R represents a hydrogen atom, or a linear or branched, saturated or unsaturated (alkyl or alkenyl), C₁-C₆ hydrocarbon-based radical;
- R' represents a linear or branched, saturated or unsaturated (alkyl or alkenyl), C₁-C₁₈ hydrocarbon-based radical, optionally substituted with a hydroxyl group;
- C-X represents C=O or CH-OH.

2. Process according to Claim 1, in which the compounds of formula (I) are chosen from those for which:
R represents H, methyl or ethyl,
R' represents a saturated C₁-C₆, or unsaturated C₂-C₆, linear hydrocarbon-based radical, optionally substituted with a hydroxyl group.

3. Process according to Claim 1 or 2, in which the compounds of formula (I) are chosen from those for which:
- when -C-X represents C=O, R denotes hydrogen and R' represents a linear C₁-C₆ alkyl radical, optionally substituted with an OH, and more preferentially R' denotes methyl or ethyl; or
- when -C-X represents CH-OH, R denotes hydrogen and R' represents a linear C₁-C₆ alkyl radical, optionally substituted with an OH, and more preferentially R' denotes methyl or ethyl.

4. Process according to any one of Claims 1 to 3, in which the compounds of formula (I) are chosen from compounds (1), (2) and (3) below:

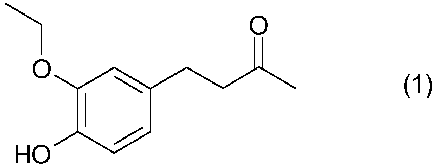

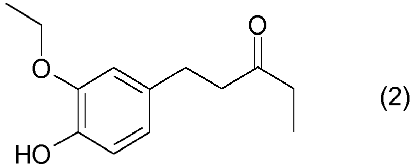

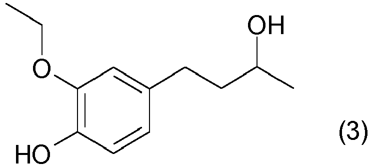

5. Process according to Claim 4, in which the compound of formula (I) is compound (1) of structure:

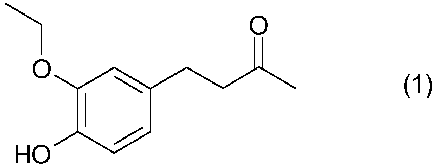

6. Process according to any one of Claims 1 to 4, in which the compound (s) of formula (I) are present in concentrations ranging from 0.01% to 10% by weight, more preferentially from 0.5% to 5% and even more preferentially from 1% to 3% by weight relative to the total weight of the composition.

7. Process according to any one of Claims 1 to 6, in which the composition does not comprise any essential oil.

8. Process according to any one of Claims 1 to 6, in which the composition is different from a composition comprising at least one essential oil and from 0.5% to 5% by weight of at least one 4-(3-ethoxy-4-hydroxyphenyl)alkyl ketone compound of formula (I) in which -C-X represents C=O.

9. Cosmetic use of at least one compound of formula (I) according to any one of Claims 1 to 5, as a deodorant active agent.

10. Use according to Claim 9, in which the compound of formula (I) is contained in a composition comprising a cosmetically acceptable medium.

11. Use according to Claim 9 or 10, in which the composition does not comprise any essential oil.

12. Use according to Claim 9 or 10, in which the composition is different from a composition comprising at least one essential oil and from 0.5% to 5% by weight of at least one 4-(3-ethoxy-4-hydroxyphenyl)alkyl ketone compound of formula (I) in which -C-X represents C=O.

13. Composition conditioned
(i) in pressurized form in an aerosol device or in a pump-dispenser bottle;
(ii) in a device equipped with a perforated wall, especially a grate;
(iii) in a device equipped with a ball applicator ("roll-on");
(iv) in the form of a wand (stick);
(v) in the form of a loose or compacted powder, **characterized in that** it contains, in a cosmetically acceptable medium, at least one compound of formula (I) as defined in any one of Claims 1 to 5;
said composition also comprising at least one antiperspirant salt or complex and/or at least one additional deodorant active agent.

14. Composition according to Claim 13, **characterized in that** it does not comprise any essential oil.

15. Composition according to Claim 13, **characterized in that** it is different from a composition comprising at least one essential oil and from 0.5% to 5% by weight of at least one 4-(3-ethoxy-4-hydroxyphenyl)alkyl ketone compound of formula (I) in which -C-X represents C=O.

16. Composition, **characterized in that** it contains, in a cosmetically acceptable medium, at least one compound of formula (I) as defined in any one of Claims 1 to 5 and at least one antiperspirant salt or complex and/or at least one additional deodorant active agent.

17. Composition according to Claim 16, **characterized in that** it does not comprise any essential oil.

18. Composition according to Claim 16, **characterized in that** it is different from a composition comprising at least one essential oil and from 0.5% to 5% by weight of at least one 4-(3-ethoxy-4-hydroxyphenyl)alkyl ketone compound of formula (I) in which -C-X represents C=O.
